(19) Europäisches Patentamt

European Patent Office

Office européen des brevets

(11) **EP 4 321 900 A1**

(12) **EUROPEAN PATENT APPLICATION**

(43) Date of publication:
**14.02.2024 Bulletin 2024/07**

(21) Application number: **22315181.2**

(22) Date of filing: **10.08.2022**

(51) International Patent Classification (IPC):
*G01S 7/52* (2006.01)     *G01S 15/89* (2006.01)
*A61B 8/08* (2006.01)     *G10K 11/34* (2006.01)

(52) Cooperative Patent Classification (CPC):
**G01S 7/52046; G01S 7/52026; G01S 15/8915;**
**G10K 11/346;** B06B 2201/20

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **SUPERSONIC IMAGINE**
**13290 Aix en Provence (FR)**

(72) Inventors:
• **Frappart, Thomas**
 **13090 Aix-en-Provence (FR)**
• **Regnier, Louise**
 **13290 Aix-en-Provence (FR)**

(74) Representative: **Paustian & Partner Patentanwälte**
 **mbB**
 **Oberanger 32**
 **80331 München (DE)**

(54) **METHOD AND SYSTEM FOR OPTIMIZING A PROCESS FOR CONSTRUCTING ULTRASOUND IMAGE DATA OF A MEDIUM**

(57) The invention relates to a method of optimizing a process for constructing ultrasound image data of a medium, wherein the method comprises:

providing (a) ultrasound spatio-temporal signal data of the medium,

determining (c) a specular property of the medium as a function of the signal data,

optimizing (d) the process based on the specular property.

Fig. 2

**Description**

BACKGROUND

It is known to use a plurality of transducer elements or transceivers (for example arranged as an array) for communication, imaging or scanning purposes, for example in the field of medical imaging, radar, sonar, seismology, wireless communications, radio astronomy, acoustics and biomedicine. One example comprises ultrasound imaging.

The aim of ultrasound imaging is to estimate the medium reflectivity. In a • conventional ultrasound imaging method, an ultrasound transducer device (also referred to as an ultrasound probe) with a set of ultrasound transducer elements may be used. In the method, one or multiple transducers are used to transmit one or successively several ultrasound beam into a medium, corresponding to a transmission operation. Then, in a reception operation a set of backscattered echo signals are received from the medium by the same set or another set of transducer elements. In particular, each of the transducer elements converts a received echo signal into for example an electrical signal. The signal may further be processed by the ultrasound system or by any associated (dedicated) system. For example, they may be amplified, filtered digitalized and/or a signal conditioning operation may be carried out. The transducer elements may be arranged as a transducer line or as a transducer array or any other configuration.

Conventionally, said signals are then transmitted to an image processing system. The received signals may be processed to generate image data of the scanned medium, for example using a beamforming method, more in particular a delay and sum (DAS) beamforming method. Generally, beamforming may be understood as a signal processing technique conventionally used in sensor arrays for directional signal transmission and/or reception. This process is used to generate beamformed data. In other words: beamforming may be understood as a signal processing technique that is achieved by combining elements-in an antenna array (for example an ultrasound transducer) in such a way that the combined signals form constructive interferences.

SUMMARY OF THE DISCLOSURE

The system and method described herein are related to technologies for optimizing a process for constructing ultrasound image data of a medium, which can improve the quality of the ultrasound image data, and which is advantageously fast and/or computationally efficient. For example, it is desirable that the method and system allow an image data construction at a predefined imaging framerate, for example at 50 Hz. In other words, the method and system desirably do not cause a significant delay in the image data construction process and may for example allow a real-time or at least quasi real-time imaging. In addition, it is desirable that the method and system can achieve an improved quality of the ultrasound image data and at the same time reduce the number of ultrasound beams transmitted into the medium.

Therefore, a method of optimizing a process for constructing ultrasound image data of a medium is provided. The method comprises:

> providing ultrasound spatio-temporal signal data of the medium,
> determining a specular property of the medium as a function of the signal data,
> optimizing the imaging process based on the specular property.

By providing such a method, it becomes possible to improve the quality of the ultrasound image data and in a fast and/or computationally efficient manner. In particular, the method allows an improvement of a conventional delay and sum (DAS) beamforming process.

More in particular, the method of the present disclosure may allow that specular properties of the medium can be taken into account in the image construction process, and for example specular reflectors in the medium can be detected. As a consequence, the process for constructing image data can be adapted and thus optimized respectively. Such an adaptive image construction can not only improve the quality of the image data but also reduce the number of required ultrasound beams transmitted into the medium.

For example, the method of the present disclosure can lead to a better imaging of specular reflectors.

Moreover, the method of the present disclosure can be fast and/or computationally efficient, as it determines the specular property of the medium directly on the spatio-temporal (i.e. pre-beamformed or "raw") signal data received from the medium (e.g. using a transducer element). Hence, no further processing of said spatio-temporal signal data is required.

Determining the specular property may comprise:

> determining for a spatial region in the medium a probability of presence of a specular reflector and/or a specular reflector angle of the specular reflector, and

constructing a specular property map based on the determined probability and/or specular reflector angle.

Furthermore, determining the specular property may comprise: determining for each of a plurality of spatial regions in the medium a probability of presence of a specular reflector and/or a specular reflector angle of the specular reflector, and

constructing a specular property map based on the determined probabilities and/or specular reflector angles.

The spatio-temporal signal data may comprise at least one of: radio-frequency, RF, signal data (for example modulated RF signal data), in-phase, quadrature-phase, IQ, demodulated data,

pre-beamformed ultrasound data,

multichannel signal data and/or per-channel signal data, signal data obtained by a transducer device comprising a plurality of transducer elements, wherein the output of each transducer element forms a channel of the signal data.

The process may be pre-defined in all or in part.

The process may comprise a beamforming process, for example a Delay and Sum (DAS) beamforming process. Ultrasound signal data of the medium may be beamformed to obtain image data of the medium.

The process may be a B-mode imaging process or synthetic B-mode imaging process. For example, a synthetic ultrasound imaging method is known from EP2101191 (A2) filed by the applicant.

The image data may be B-mode image data.

The signal data used in the process and/or for beamforming may at least partially correspond to the signal data used for determining the specular property. This may for example be the case for synthetic B-mode where plane waves are emitted and the collected RF data may be used for both, B-mode imaging, and specular determination.

The process and/or the beamforming process may comprise: determining at least one subset of signal data as a function of a predefined spatial region in the medium, and

determining image data of the spatial region is based on the subset.

The subset may for example be determined by a Delay and Sum (DAS) beamforming method.

The image data of the spatial region may be for example a pixel or a voxel.

The spatial regions represented by the subsets may correspond to the spatial regions represented by the probabilities of presence of a specular reflector. However, they may also differ from each other, for example by having a higher or lower resolution. In this case, it is desirable to provide a mapping between the two types of spatial regions.

The subset may comprise a spatial and temporal selection of signal data, i.e. a selection which comprises a spatial and a temporal component. In other words, the subset may be selected from the RF signal data as a function of a used DAS beamforming method.

The spatial selection, i.e. the spatial component of the spatial and temporal selection, may define a receive aperture and/or a selection of channels used for forming the subset.

The temporal selection, i.e. the temporal component of the spatial and temporal selection, may be based on a predefined delay algorithm (for example defined by the used DAS beamforming method). Accordingly, the selected channel data may have a specific delay to each other according to the used DAS beamforming method.

The spatial and temporal selection may be further determined as a function of an estimated receive angle associated with a response signal received from the predefined spatial region.

The spatial and temporal selection may be adapted by changing the receive angle as a function of the determined specular reflector angle of a specular reflector identified at the spatial region.

Accordingly, meanwhile in a conventional (DAS) beamforming method the angle of the transmitted beam may be estimated to be equal to the receive angle (for example 0°, i.e. perpendicular to the transducer array of the used transducer element), in the method of the present disclosure the estimated receive angle may advantageously be different to the transmit angle.

The spatial and temporal selection may be adapted by changing (i.e. shifting) and/or limiting the spatial selection as a function of the specular property.

The spatial and temporal selection may be further determined as a function of a receive aperture defined by a selection of channels used to receive a response signal from the predefined spatial region.

The spatial selection may be changed and/or limited by respectively changing and/or reducing the aperture. Accordingly, the aperture may be defined by the number of used channels.

A channel may correspond to one transducer element of a transducer device, which may comprise an array of transducer elements.

Conventionally, the number of channels used to receive a response signal may be predefined. Their relative position with regard to the transducer array may be determined as a function of the spatial region from which image data shall be collected. In particular, the relative position is conventionally chosen such that the selected channels are directly in front of the spatial region (i.e. the receive angle is 0°, i.e. not tilted). However, according to the present disclosure this relative position and optionally also the number of selected channels may be adaptive (i.e. adapted, i.e. changed) as a function of the determined specular property (in particular, in case a specular reflector has been detected at the region).

The process may be optimized for the complete image (i.e. all scanned regions). However, it is also possible that the process is optimized only for those subsets which are associated with a spatial region having a probability of presence of a specular reflector above a predefined threshold. In other words, only those scanned regions, where a specular reflector has been detected, may be optimized.

The ultrasound signal data of the medium may be associated to a plurality of ultrasound waves emitted in the medium, or at least one ultrasound wave emitted in the medium.

The emitted ultrasound waves may comprise non-focalized and/or plane waves having different emission angles.

Accordingly, as plane waves may be used to determine the specular properties, in particular the specular angles, the number of required ultrasound beams transmitted into the medium may be reduced. In other words, less transmissions of emission waves are required to cover a given angle specular range. Hence, less transmissions are necessary and therefore a delay caused by the method of the present disclosure, i.e. its impact on an imaging framerate, can advantageously be reduced. In other words, more information from the medium can be acquired in less time.

In more detail, many specular reflectors like biopsy needles have a plane wave acoustic signature in the backscattered echoes when illuminated by an incident plane wave. This symmetry between the transmit beam and the received acoustic signals can be advantageously used to efficiently compute the specular angle of the reflector. Accordingly, the method can become computationally more efficient.

Furthermore, it may indeed be desirable to have in addition a focused B-mode for image quality reasons. That is why, having a duplex sequence (of planes waves and focalized waves) may be advantageous. Accordingly, using plane waves in combination with focused B-mode has the advantage of sampling the whole space and specular angles in less time than using focused beams. Accordingly, more information can be acquired in less time.

Furthermore, providing ultrasound signal data may comprise: transmitting an emitted sequence of ultrasound waves into the medium, and receiving a response sequence of ultrasound waves from the medium, wherein the ultrasound signal data are based on the response sequence of ultrasound waves. Alternatively, ultrasound signal data may be provided by a data interface and/or a data storage.

The response sequence may also be understood as a response signal from the spatial region.

The emitted sequence may be transmitted using an ultrasound transducer device comprising a plurality of ultrasound transducer elements.

The response sequence may be received using another or the same ultrasound transducer device.

At least in synthetic B-mode imaging, the provided ultrasound signal data may be used for both determination of specular property and for constructing the B-mode image.

However, it is generally possible that for the process of constructing ultrasound image data, additional signal data is provided. Accordingly, the process may additionally comprise operations of:

transmitting an emitted sequence of ultrasound waves (for example focalized waves) into the medium, and receiving a response sequence of ultrasound waves from the medium.

Optimizing the process may comprise: adapting the transmission and/or reception as a function of the specular property.

Accordingly, the physical signal acquisition process may be adapted as a function of detected reflectors in the medium. For example, the emitted sequence of ultrasound waves may be adapted. For instance, in case plane waves are emitted, their emission angles may be adapted such that the received response sequence comprises ultrasound RF data of the detected specular reflector. However, even if the emission angle is not adaptive but predefined, the present disclosure still allows adapting the receive aperture of the used transducer device, as explained above. For example, the tilt angle of emitted plane waves in synthetic B-mode may be adapted.

The present disclosure may further relate to a method of constructing ultrasound image data of a medium. The method may comprise:

a method of optimizing a process for constructing ultrasound image data of a medium, as described above, and constructing ultrasound image data of the medium using the optimized process.

The present disclosure may also refer to a computer program comprising computer-readable instructions which when executed by a data processing system cause the data processing system to carry out a method according to any examples of the present disclosure. In case the method operations may comprise any (physical) aspects which go beyond a mere data processing (for example an ultrasound wave emission), the computer program may further comprise computer-readable instructions which when executed by a data processing system cause any external elements of a system (for example an ultrasound transducer device) to carry out these operations.

The disclosure may refer to a system for optimizing an ultrasound process for constructing ultrasound image data of a medium. The system comprises a processing unit configured to: receive ultrasound spatio-temporal signal data of the medium, determine a specular property of the medium as a function of the signal data, optimize the process based on the specular property.

The system may for example comprise or be connectable to a transducer device which acquires the ultrasound spatio-temporal signal data. It is further possible that the system receives the ultrasound spatio-temporal signal data from an external data system, e.g. a data storage.

The system may furthermore be configured to perform any of the method features or operations described above.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification, illustrate examples of the disclosure and together with the description, and serve to support and illustrate the principles thereof.

BRIEF DESCRIPTION OF THE DRAWINGS

Fig. 1 shows a schematic drawing of an ultrasound system according to examples of the present disclosure.
Fig. 2 shows a flowchart of a method of optimizing a process for constructing ultrasound image data of a medium according to the present disclosure.
Fig. 3 shows an exemplary reflection of a plane wave of a specular reflector in a medium.
Fig. 4 shows an IQ interpolation of an exemplary ultrasound wave front for the Snell transform.
Fig. 5 shows an exemplary Snell matrix (left diagram) and simulated and theoretical Snell transform for $\gamma 0=10°$ reflector (right diagram).
Fig. 6 shows an example of a conventionally reconstructed image of a Rayleigh scatterer (left) and the associated spatio-temporal signal data (right).
Fig. 7 shows an example of a conventionally reconstructed image of a Rayleigh scatterer and a specular reflector (left) and the associated spatio-temporal signal data (right).
Fig. 8 shows the example of fig. 7, wherein the receive angle is adjusted according to the tilt angle of the specular reflector.
Fig. 9 shows the example of fig. 7 and fig. 8, wherein the receive angle is adjusted according to the tilt angle of the specular reflector, but only for the pixels illustrating the specular reflector.

DESCRIPTION OF THE DRAWINGS

Reference will now be made in detail to examples of the disclosure, examples of which are illustrated in the accompanying drawings. Wherever possible, the same reference numbers will be used throughout the drawings to refer to the same or like parts..

In general, conventional beamforming processes assume the received signals are backscattered echoes from Rayleigh diffusors (i.e. whose typical sizes are smaller than the transmitted pulse wavelength).

However, many reflectors inside for example a human body are showing a specular behavior such as tendons, muscle fibers, interfaces, etc. This means that, for those reflectors, the direction of backscattered acoustic field main lobe depends on the Snell-Descartes law. Basically, it varies with the specular reflector angle. As a consequence, the resulting image quality obtained by the image processing system can be deteriorated by such reflectors being in the studied region.

Rodriguez-Molares et.al. describes an adaptive beamforming technique that takes into account the physics of specular reflection. Specular patterns, predicted by Snell's law of reflection, are detected across the pool of received data and used to enhance the visualization of specular interfaces, cf. for example:

Rodriguez-Molares, A., Fatemi, A., Torp, H., & Løvstakken, L. (2016, September). Adaptive beamforming based on Snell's law of reflection. In 2016 IEEE International Ultrasonics Symposium (IUS) (pp. 1-4). IEEE, and
Rodriguez-Molares, A., Fatemi, A., Løvstakken, L., & Torp, H. (2017). Specular beamforming. IEEE Transactions on Ultrasonics, Ferroelectrics, and Frequency Control, 64(9), 1285-1297.

The system and method described herein are related to technologies for optimizing a process for constructing ultrasound image data of a medium, in particular in the context of medical imaging. The method is in particular suitable for processing signal data of a medium scanned by a transducer device. For example, the method may be used in a device such as for instance an ultrasound system.

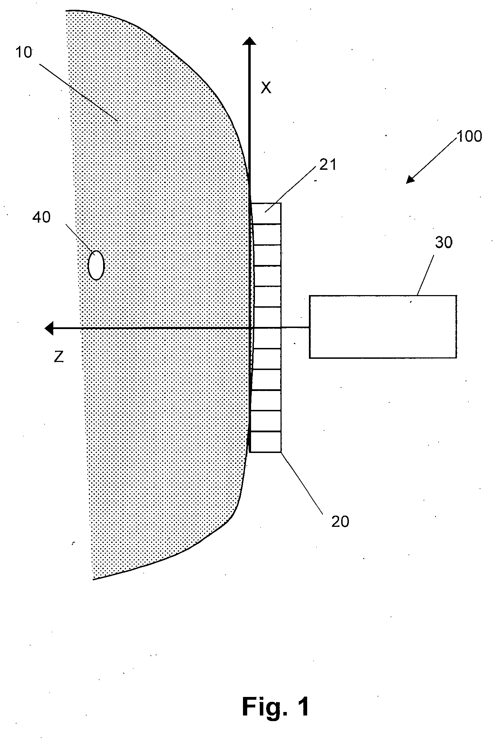

Fig. 1 shows a schematic drawing of an ultrasound system 100 according to examples of the present disclosure. The system 100 shown on Fig. 1 is adapted for collecting ultrasound spatio-temporal signal data of the medium 10, for instance living tissues and/or in particular human tissues of a person. The system 100 is further configured to construct ultrasound spatio-temporal signal data of the medium 10 in an optimized manner by taking specular properties of the medium into account. The system may include for instance:

- A plurality of transducer elements 21. The transducer elements may be configured to transmit (a) a pulse into the medium and/or to receive (b) a plurality of signals from the medium, optionally in response to transmitting (a) the pulse into the medium. A transducer array comprising a plurality of transducer elements 21 may be used. For example, a linear array 20 may be provided typically including a few tens of transducer elements (for instance 100 to 300) juxtaposed along an axis X (horizontal or array direction X) as already known in usual probes. In this example, the array 20 is adapted to perform a bidimensional (2D) imaging of the medium 10, but the array 20 could also be a bidimensional array adapted to perform a 3D imaging of the medium 10. Accordingly, a matrix of transducers may be used. It is though also possible that the system comprises a single line of transducers moveable in a probe, such that 3D imaging can be achieved. The transducer array 20 may also be a convex array including a plurality of transducer elements aligned along a curved line. The same transducer element(s) may be used to transmit a pulse and receive the response, or different transducer elements are used for transmission and reception. There may be one or more emitting transducer elements and a plurality of receiving transducer elements. In a further alternative, only a single transducer element may be used which receives a plurality of signals which have different spatial properties (for example originating from different spatial regions). The transducer element may for instance be electronically or physically moveable;

- an electronic control device 30 controlling the transducer array and acquiring signals therefrom. The electronic control device 30 may be part of a probe which also comprises the transducer elements 21. Alternatively, the electronic control device 30 may be external to the probe, or consist of several devices which are partially part of the probe and partially external thereto.

The system may further include a processing unit (not shown) for controlling the electronic control device 30 and/or for example for sending data to an external device, such as for example, a server, a computer on which an artificial intelligence (AI) algorithm is running, a dedicated workstation, presenting data, a device for displaying images obtained from the electronic control device or any of the other external devices. Accordingly, the method according to the present disclosure, in particular a method for optimizing a process for constructing ultrasound image data, may be carried out by at least one of the electronic control device 30, the processing unit or any of the external devices. Furthermore, the process for constructing the ultrasound image data may be carried out by the same processing device as that one for optimizing the optimizing a process, or (at least in part) by another one.

According to further examples, the system 100 may include at least one processing unit (or processor) and memory. In examples, the processor and memory unit may be incorporated into the system such as depicted in FIG. 1 or may be a computer or computer communicatively linked thereto. Depending on the exact configuration and type of computing device, memory (storing, instructions to evaluate ultrasound data or otherwise perform the methods described herein) may be volatile (such as RAM), non-volatile (such as RAM, flash memory, etc.), or some combination of the two. Further, the system 100 may also include storage devices (removable and/or non-removable) including, but not limited to, magnetic or optical disks or tape. Similarly, the system 100 may also have input device(s) such as keyboard, mouse, pen, voice input, etc. and/or output device(s) such as a display, speakers, printer, etc. Also included in the environment may be one or more communication connections, such as LAN, WAN, point to point, etc. In embodiments, the connections may be operable to facility point-to-point communications, connection-oriented communications, connectionless communications, etc.

The system 100 typically includes at least some form of computer readable media. Computer readable media can be any available media that can be accessed by processing unit (or processor) or other devices comprising the operating environment. By way of example, and not limitation, computer readable media may comprise computer storage media and communication media. Computer storage media includes volatile and nonvolatile, removable and non-removable media implemented in any method or technology for storage of information such as computer readable instructions, data structures, program modules or other data. Computer storage media does not include communication media.

Communication media embodies computer readable instructions, data structures, program modules, or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared, microwave, and other wireless media. Combinations of the any of the above should also be included within the scope of computer readable media.

The system 100 may be a single computer operating in a networked environment using logical connections to one or more remote computers. The remote computer may be a personal computer, a server, a router, a network PC, a peer device or other common network node, and typically includes many or all of the elements described above as well as others not so mentioned. The logical connections may include any method supported by available communications media. Such networking environments are commonplace in offices, enterprise-wide computer networks, intranets and the Internet.

The transducer elements 21 may comprise piezo-crystals and/or other components that may be configured to generate and/or record and/or receive signals. The terms transducer and transducer elements may be used synonymously throughout this disclosure unless denoted differently.

Transducer elements 21 may be configured to generate and/or record and/or receive signals, optionally ultrasonic signals. Transducer elements 21 and/or electronic control device 30 and/or the processing unit may be configured to determine phase properties of spatio-temporal signal data.

The axis Z on figure 1 is an axis perpendicular to the axis X, for example in the depth direction of the examined medium. This direction is designated in the present document as a vertical or axial direction. An ultrasound beam emitted in the direction of the Z-axis may be understood as having a tilt angle of 0°.

The medium 10 may comprise a spatial region 40. The spatial region 40 may constitute a specular reflector in the medium, as described in more detail in the following examples according to the present disclosure. The medium 10 may comprise a plurality of such spatial regions 40.

The system herein disclosed is a device for ultrasound imaging, the transducer elements are ultrasound transducer elements, and the implemented method evaluates an assumed wave propagation speed in the medium 10 based on phase properties of spatio-temporal signal data. The medium 10 is associated with the spatio-temporal signal data. The method optionally may produce ultrasound images of the medium 10 and/or of spatial regions 40 of the medium 10 and/or may send data to a dedicated server or working station.

However, the system may be any imaging or sensor device using other waves than ultrasound waves (for example waves having a wavelength different than an ultrasound wavelength and/or waves being no sound waves), the transducer elements and the electronic control device components and related elements being then adapted to said waves.

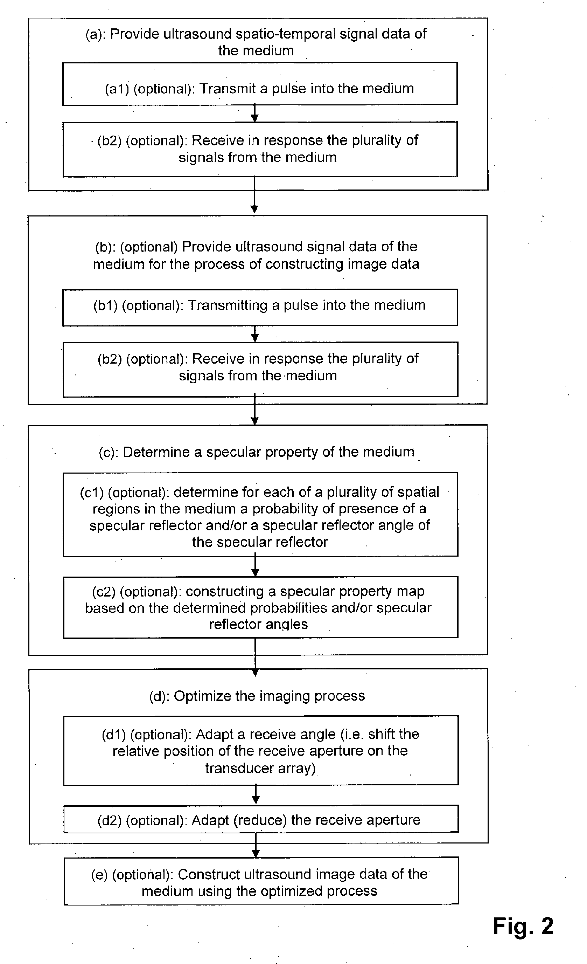

Fig. 2 shows a flowchart of a method of optimizing a process for constructing ultrasound image data of a medium according to the present disclosure. The method may be implemented in the system of Fig. 1.

The method comprises an operation (a) of providing ultrasound spatio-temporal signal data of the medium. The spatio-temporal signal data may be pre-beamformed ultrasound data, i.e. "raw" multi-channel signal data obtained by a transducer device comprising a plurality of transducer elements, wherein the output of each transducer element forms a channel of the signal data.

For example, said operation (a) may comprise an optional operation (a1) of transmitting a pulse into the medium. For example, the transmission operation may comprise insonification of the medium with a one or several plane waves (i.e. non-focalized waves). The plane wave may have different phases, i.e. different angles. More in particular, during the transmission operation a plurality of ultrasonic waves may be transmitted into a spatial region 40. For example, the phase (i.e. the delay) of each transmitted pulse (of each respective transducer element) may be adjusted to shape the transmit wavefront. To get different angles, the transmit delay law (hence the phase shift) may be proportional to the sine of the transmit angle.

Generally, in the present disclosure a pulse may correspond to an acoustic and/or electrical signal emitted by a transducer element. The pulse may for example be defined by at least one of: the pulse duration, the frequency of the resulting wave, the number of cycles at the given frequency, the polarity of the pulse, etc. A wave may correspond to the wavefront generated by one or several transducer elements (i.e. by respectively emitted pulses). The wave may be controlled by means of emission delay between the different used transducer elements. Examples comprise a plane wave, a focused wave and a divergent wave. A beam may correspond to the physical area insonified by the wave (for example in the medium). Hence, the beam may be related to the wave but may have less or no temporal notion. For example, it may be referred to a beam when the depth of field of a focused beam is of interest.

In an optional operation (a2), a plurality of signals may be received, optionally in response, from the medium by the plurality 20 of transducer elements 21. The plurality of signals may comprise backscattered echoes of the transmission of operation (a1). The response sequence may also be referred to as spatio-temporal data and/or signal data, in particular ultrasound signal data and/or RF and/or IQ signal data (i.e. this data may correspond to the spatio-temporal signal data of operation (a)). The signal data may be in the time domain, more in particular in a spatio-temporal domain, as for example described in more detail below. In one example, the response sequence may be processed by bandpass filtering, in order to keep only one or several frequency ranges.

It is noted that operations (a1) to (a2) are optional, as they may also be carried out by any other system and/or at another time. Data may also be provided in operation (a) by an external system, etc. It is also possible that the spatio-temporal signal data are pre-stored, and for example provided by/read on a data storage, a communication interface, etc.

In an optional operation (b) ultrasound signal data of the medium for the process of constructing image data. Accordingly, it is possible that different ultrasound signal data are used for image construction that those provided in operation (a) which are used to determine a specular property (cf. operation (c)). It is however also possible that the same ultrasound signal data are used for both purposes, in particular in case the image construction process comprises a synthetic B-mode imaging process.

The optional operation (b) may in particular comprise an optional operation of transmitting (b1) a pulse into the medium. For example, the transmission operation may comprise insonification of the medium with a one or several focalized waves. The waves may be focalized on different regions in the medium comprising the spatial region 40. Using focalized waves may for example be advantageous in case of a conventional B-mode image construction process.

In an optional operation (b2), a plurality of signals may be received, optionally in response, from the medium by the plurality 20 of transducer elements 21. The plurality of signals may comprise backscattered echoes of the transmission of operation (b1). The response sequence may also be referred to as spatio-temporal data and/or signal data, in particular ultrasound signal data and/or RF and/or IQ signal data. The signal data may be in the time domain, more in particular in a spatio-temporal domain, as for example described in more detail below. In one example, the response sequence may be processed by bandpass filtering, in order to keep only one or several frequency ranges.

It is noted that operations (b1) to (b2) are optional, as they may also be carried out by any other system and/or at another time. Data may also be provided in operation (b) by an external system, etc. It is also possible that the signal data are pre-stored, and for example provided by/read on a data storage, a communication interface, etc.

In operation (c) a specular property of the medium is optimized as a function of the signal data provided in operation (a).

Operation (c) may comprise an optional operation (c1), in which for each of a plurality of spatial regions in the medium (or at least for the one spatial region 40) a probability of presence of a specular reflector and/or a specular reflector angle of the specular reflector is determined. The specular reflector angle may only be determined, in case the probability of presence of the respective specular reflector is determined to exceed a predefined minimum threshold.

Operation (c) may comprise a further optional operation (c2), in which a specular property map is constructed based on the determined probabilities and/or specular reflector angles of the plurality of regions. In other words, the specular property map may indicate for each region the respective specular reflector and/or a specular reflector angle.

Operation (d) may comprise an optional operation (d1), in which a (predefined) receive angle used in the process is adapted (i.e. changed). Accordingly, the relative position of the receive aperture on the transducer array may be shifted. Moreover, in a further optional operation (d2) the receive aperture is adapted, in particular reduced.

In optional operation (e), the process for constructing ultrasound image data is optimized based on the determined specular property or optionally based on the specular property map. Said process may be basically pre-defined but may be adaptable (i.e. adaptive) by means of operation (d).

For example, the process may comprise a beamforming process. For instance, the process may be configured to beamform ultrasound signal data of the medium obtained in operation (a) and/or operation (b) to obtain image data of the medium. More in particular, the process may be a B-mode imaging process or synthetic B-mode imaging process.

Generally, the beamforming process and thus the construction process may comprise: determining at least one subset of signal data as a function of a predefined spatial region in the medium, and determining image data of the spatial region is based on the subset. The subset may for example be determined by a Delay and Sum (DAS) beamforming method. The image data of one spatial region may be for example a pixel or a voxel.

The spatial regions represented by the subsets may correspond to the spatial regions represented by the specular property map. However, they may also differ from each other, for example by having a higher or lower resolution. In this case, it is desirable to provide a mapping between the at least two types of spatial regions.

The subset may comprise a spatial and temporal selection of signal data. In other words, the subset may be selected from the RF signal data as a function of a used DAS beamforming method.

According to a further aspect, the spatial selection may define a (predefined but adaptable) receive aperture and/or a selection of channels used for forming the subset. For example, the temporal selection may be based on a predefined delay algorithm (for example defined by the used DAS beamforming method). Accordingly, the selected channel data may have a specific predefined delay to each other according to the used DAS beamforming method. The spatial and temporal selection may be further determined as a function of an estimated (predefined) receive angle associated with a response signal received from the predefined spatial region.

The spatial and temporal selection may be adapted by changing the receive angle as a function of the determined specular reflector angle of a specular reflector identified at the spatial region.

Accordingly, operation (d) may comprise an optional operation (d1), in which the (predefined) receive angle used in the process is adapted (i.e. changed).

As a consequence, meanwhile in a conventional (DAS) beamforming method the angle of the transmitted beam may be estimated to be equal to the receive angle (for example 0°, i.e. perpendicular to the transducer array of the used transducer element), in the method of the present disclosure the estimated receive angle may be different to the transmit angle.

The spatial and temporal selection may be further determined as a function of a receive aperture defined by a selection of channels used to receive a response signal from the predefined spatial region.

The aperture may be defined by the number of used channels. A channel may correspond to one transducer element of a transducer device, which comprises an array of transducer elements. Conventionally, the number of channels used to receive a response signal may be predefined. Their relative position with regard to the transducer array may be determined as a function of the spatial region from which image data shall be collected. In particular, the relative position is conventionally chosen such that the selected channels are directly in front of the spatial region (i.e. the receive angle is 0°, i.e. not tilted).

However, according to the present disclosure, this relative position and optionally also the number of selected channels may be adapted (i.e. changed) as a function of the determined specular property ; in particular, in case a specular reflector has been detected at the region.

Furthermore, according to the present disclosure, the spatial selection, i.e. the spatial component of the spatial and temporal selection, may be changed and/or' limited by respectively changing and/or reducing the aperture. In particular, the spatial selection may be reduced to that part (i.e. those channels) which represents echoes received from the specular reflector; Other channel data (i.e. from other channels) may be disregarded. The reason is that the signal from the other channels may not contain useful information for the reconstruction of the specular reflector.

Accordingly, operation (d) may further comprise an optional operation (d2), in which the receive aperture is adapted (i.e. limited).

Accordingly, the process may be optimized in operation (d) for the complete image (i.e. all scanned regions). However, it is also possible that the process is optimized only for those subsets which are associated with a spatial region having a probability of presence of a specular reflector above a predefined threshold. In other words, only those scanned regions, where a specular reflector has been detected, may be optimized.

As already mentioned, in optional operation (e) ultrasound image data of the medium may be constructed using the optimized process. However, it is also possible that operation (e) is omitted. For example, it is also possible that merely the process is optimized by the method. The optimized process (or information re. the optimized process) may be stored and/or provided to an external system. Said external system may then use the optimized process to construct image data of the medium.

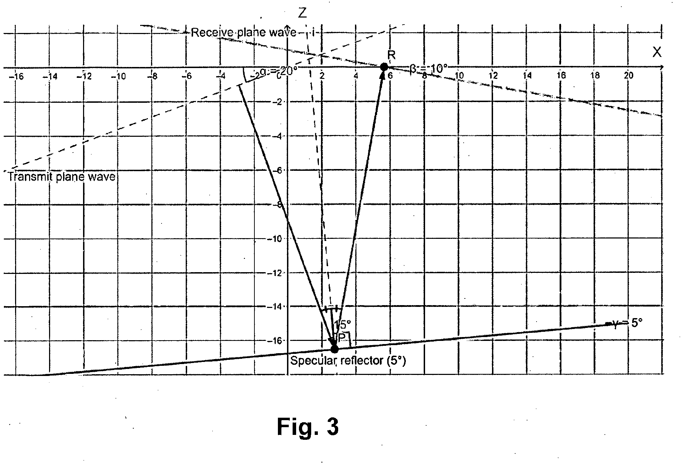

In the following, the underlying principles of the method according to the present disclosure are explained in more details, by referring to some examples in context of Fig. 3 to 9.

Delay and sum (DAS) beamforming is based on a main assumption: the dimension of each reflector in the body is smaller than the wavelength. It thus assumes they are Rayleigh diffusers. However, when the dimension of the scatterers increases compared to lambda, the scattered pressure becomes more and more angle dependent and privileged directions appear because of diffractive effects. This type of specular reflectors, such as bones, needles, fibers, tissue interfaces, are thus very badly reconstructed because they don't follow the classical hypothesis of Rayleigh diffusors of the delay-and-sum beamforming.

Such specular reflectors follow Snell's law of reflection, comparable to mirrors. The angle of the reflected plane wave depends on the angle of the reflector and the angle of the transmitted plane wave:

$$\beta = \alpha - 2\gamma \qquad \text{(eq. 1)}$$

where $\beta$ is the angle of a plane wave received from a medium,
$\alpha$ is the angle of a tilted plane waves emitted into the medium, and
$\gamma$ is the angle of the specular reflector in the medium.

Fig. 3 shows an exemplary reflection of a plane wave of a specular reflector in a medium. Similarly to fig. 1, the x-axis corresponds to the extension of an ultrasound transducer array, and the z-axis in a depth direction of the medium. In this example, a specular reflector with an angle of $\gamma$ = 5° insonified with a $\alpha$ = 20° plane wave would send a $\beta$=10° tilted plane wave back. Accordingly, meanwhile for a conventional scatterer (at the position and instead of the specular reflector P) the reception beam would be received at the emission point (i.e. at app. X=-4), the specular reflector causes the reception beam to be received at a completely different position R (i.e. at. approximately X=+6).

Therefore, the image quality in synthetic B-Mode may be affected by the presence of specular reflectors, as synthetic B-Mode uses plane waves for insonifying the medium, such as in the example of fig. 3.

However, since a plane wave that encounters a specular reflector is sent back as a plane wave to the probe, plane waves are optimal to reconstruct specular reflectors. Therefore, according to the present disclosure, instead of trying to minimize specular reflections, it is proposed to use the physics of specular reflections to build a specular beamformer, i.e. an optimized process for construction ultrasound image data which takes specular reflectors into account and is adapted correspondingly. This method allows to acquire new knowledge about the explored medium.

Exemplary aspects of the specular beamforming are described in the following. For each transmit angle, if a tilted specular reflector lies at localization $P(x, z)$, a plane wave with an angle is sent back to the probe. The forth delay transmit to point $P(x,z)$ (forth delay meaning for example the time it takes for the wavefront to get to the considered P pixel) corresponds to the classical delay law for a single plane wave, cf. eq. 2:

$$t_{transmit} = \frac{1}{c_0}(z \cos \alpha + x \sin \alpha) \qquad (eq.\ 2)$$

wherein $c_0$ is the speed of sound,
$\alpha$ is the angle of a tilted plane waves emitted into the medium,
$z$ is the distance of the specular reflector from the transducer device in the depth direction of the medium (i.e. the Z-direction being the depth dimension), and
$x$ is the distance of the specular reflector from the transducer device in the X-direction (i.e. the X-direction being the lateral dimension or the azimuth).

The backscattered plane wave is detected by the probe at localization $R(x_R, z_R = 0)$ after a return time $t_{receive}$ satisfying, cf. eq. 3 and 4:

$$t_{receive} = \frac{1}{c_0}(z \cos \beta + (x - x_R) \sin \beta) \qquad (eq.\ 3)$$

$$\tan \beta = \frac{x - x_R}{z} \qquad (eq.\ 4)$$

wherein $\beta$ is the angle of a plane wave received from a medium,
$x_R$ is the abscissa of the considered transducer element in receive mode. That is why zR=0 is at the probe.

For each pixel $P(x,z)$, a set of specular angles $\gamma$ is explored. Looking for a specular angle $\gamma$ means the receive plane wave has an angle defined by $\beta = \alpha - 2\gamma$. This step is performed for each transmit plane wave of angle $\alpha$.
Furthermore, a specular transform of the pixel $P$ may be built. The per-channel data for plane wave imaging $s(\alpha, t, x_{piezo})$ may have three dimensions: the transmitted angles, the samples or fast time t and the position of the receiving piezo corresponding to the x-coordinate. For a $\gamma_0$ tilted specular reflector at location $P$ and for each emitted plane wave, the piezo receiving the in phase wave front after a time $t_{tr} = t_{transmit} + t_{receive}$ is at location $R(x_R, z_R = 0)$, such as defined previously. The signal coming from a specular reflector at location $P$ is then, cf. eq. 5:

$$\forall \alpha, s(\alpha, t, x_{piezo}) = s(\alpha, t_{tr}, x_R) \qquad (eq.\ 5)$$

wherein $x_R$ is the position of the respective piezo in the X direction.
This signal may be built in the $(\alpha, \gamma)$ space. The specular signal may be expressed with by using the relationships between $R(x_R, 0)$ and $\beta$ and between $t_R$ and $P(x, z)$ for each transmit angle, cf. eq. 6:

$$\forall \alpha, s(\alpha, t_{tr}, x_R) = s(\alpha, P, \beta = \alpha - 2\gamma) \qquad (eq.\ 6)$$

wherin $P$ is the point (or pixel) of interest i.e. the point where it is desired to assess the probability of having a specular reflector and its angle, and
$\gamma$ is the specular angle.

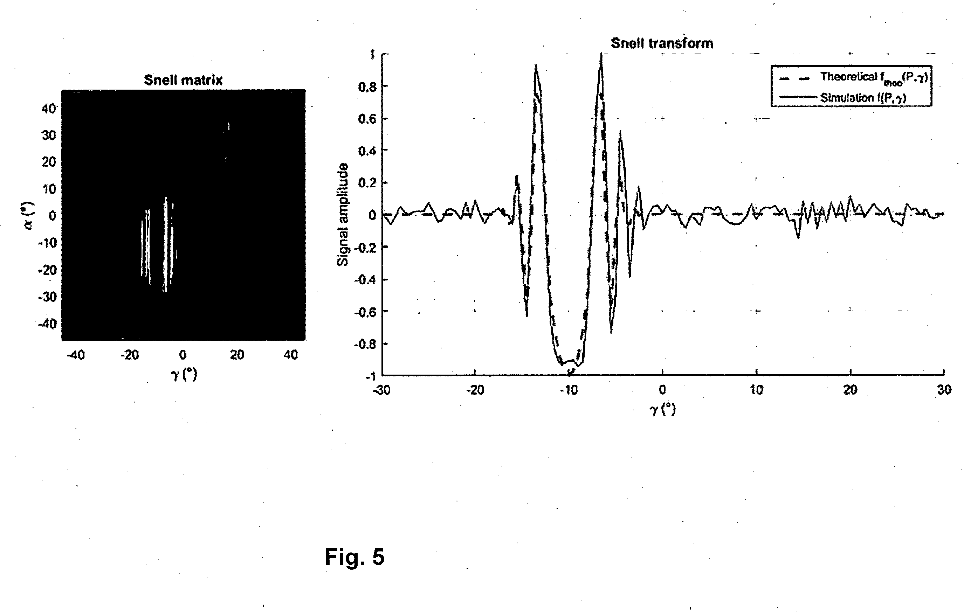

This signal $s(\alpha, P, \alpha - 2\gamma)$, displayed in the $(\alpha, \gamma)$ space may be called the Snell matrix (see an example in fig. 5, left). For each $\alpha$, the delayed echoes coming from a hypothetical $\gamma_0 = 0°$ specular angle are in phase, on the same wave front. The signal inside the matrix is maximized for $\gamma = \gamma_0$. Otherwise, there is no coherent signal in the Snell matrix if P contains only speckle.

In particular, the coherent summation of the Snell matrix may be called the specular transform, cf. eq. 7:

$$\forall P, f(P, \gamma) = \sum_{\forall \alpha} s(\alpha, P, \alpha - 2\gamma) \qquad (eq.\ 7)$$

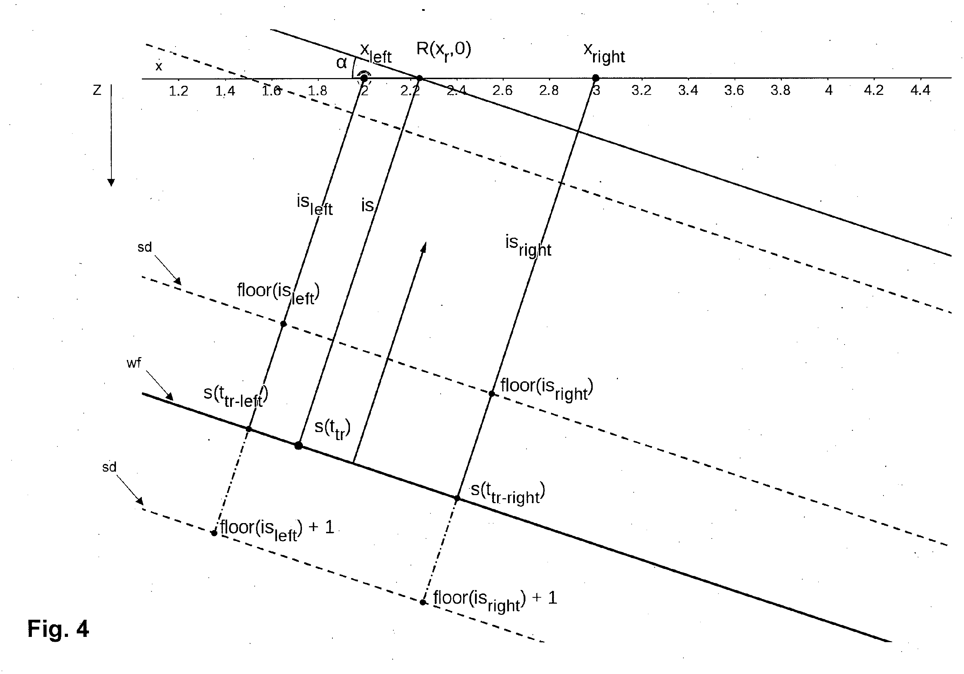

Fig. 4 shows an IQ interpolation of an exemplary ultrasound wave front for the Snell transform. Like in fig. 1 and 3, the x-axis corresponds to the extension of an ultrasound transducer array, and the z-axis in a depth direction of the medium. Fig. 3 is 90° tilted compared to Fig. 1. Vertical axis on Fig. 3 is the Z-axis (depth/axial) and horizontal axis is X-axis (lateral). Fig. 1 is thus the opposite thereto.

The specular transform may be applied to the ultrasound spatio-temporal signal data. In particular, the spatio-temporal signal data (i.e. the output data of the transducer device) may be demodulated IQ. the spatio-temporal signal data may be for example demodulated IQ or RF signals. Both may be per channel data but in the case where we demodulated IQ is used, the carrier phase needs to be taken into account during the interpolation step as explained below The interpolation part needs special attention in order to rephase the interpolated IQ data from the knowledge of the delays. The signal arriving at position $R(x_R, 0)$ after a delay $t_{tr}$ (or $is$ in sample unit) may be interpolated between the signals arriving at the nearest piezos $x_{left}$ and $x_{right}$ after a delay $t_{tr\text{-}left}$ and $t_{tr\text{-}right}$ respectively, cf. eq..8:

$$\begin{cases} t_{tr-left} = t_{tr} - \frac{1}{c_0}\sin\beta\ (x_R - x_{left}) \\ is_{left} = t_{tr-left} \cdot f_{sampling} \end{cases} \qquad (eq.\ 8)$$

The bold line **wf** corresponds to the wave front of a reflected plane wave. The dashed gray line **sd** corresponds to the sampled data in the per-channel data. The parameter "isleft" may be the exact index of the sample (i.e. not an integer) in the per channel data matrix for the transducer element whose abscissa is xleft. The parameter "floor(isleft)" may be the integer part of isleft. The parameter "isright" may be the exact index of the sample (i.e. not an integer) in the per channel data matrix for the transducer element whose abscissa is xright. The parameter "floor(xright)" may be the integer part of xright.

There may be two first interpolations for each channel:

- For channel $x_{left}$, the received signal $s_{left}$ is interpolated over samples $\lfloor is_{left} \rfloor$ and ( $\lfloor is_{left} \rfloor$ + 1). One has to take into account the phase rotation introduced by the carrier wave,
- The same operation may be performed to recover the signal $s_{right}$.

Then, the Snell transform $s(t_{tr})$ may be obtained by finally interpolating $s_{left}$ and $s_{right}$.

The specular beamforming may be used to correlate the previous built signal with a matched filter, that is the signal reflected by a plane reflector with tilt $\gamma_0$. The theoretical model of $f(P, \gamma)$ can be derived for $\gamma_0 = 0°$ and generalized with a convolution by $\delta(\gamma - \gamma_0)$. For example, from a two-way pulse $e2w(t)$ of the simulated system the theoretical may be built over. The purpose is to find the correspondence between the time delays and the characteristics of the specular reflector.

An expression of the Snell transform may be derived from a specular reflector of angle $\gamma_0 = 0°$, knowing the two-way impulse response, cf. eq. 9:

$$f_{theo}(P, \gamma) = \sum_{\alpha} s_{theo}(\alpha, P, \gamma) = \sum_{\alpha} e_{2w}\left(\frac{z}{c}(\cos 2\gamma + \sin 2\gamma \tan 2\gamma - 1)\right) \qquad (eq.\ 9)$$

Fig. 5 shows an exemplary Snell matrix (left diagram) and simulated and theoretical Snell transform for $\gamma0=10°$ reflector (right diagram). In particular, the right diagram of fig. 5 shows both the simulated and the theoretical Snell transform for an exemplary $\gamma_0 = -10°$ tilted reflector. The Snell matrix is actually symmetric with respect to $\gamma = \gamma_0$. The

theoretical signal is convoluted by $\delta(\gamma - \gamma_0)$ to retrieve the signal for any value of $\gamma_0$.

For each pixel $P$, the normalized correlation $r(P, \gamma)$ between the Snell transform $f(P, \gamma)$ and the theoretical $f_{theo}$ is computed. It gives the likelihood of having a specular reflector at the location P, and its angle, cf. eq. 10:

$$\begin{cases} \Lambda(P,\gamma) = \max\{r(P,\gamma)\} \\ \gamma_0(P) = arg\langle\max\{r(P,\gamma)\}\rangle \end{cases} \qquad \text{(eq. 10)}$$

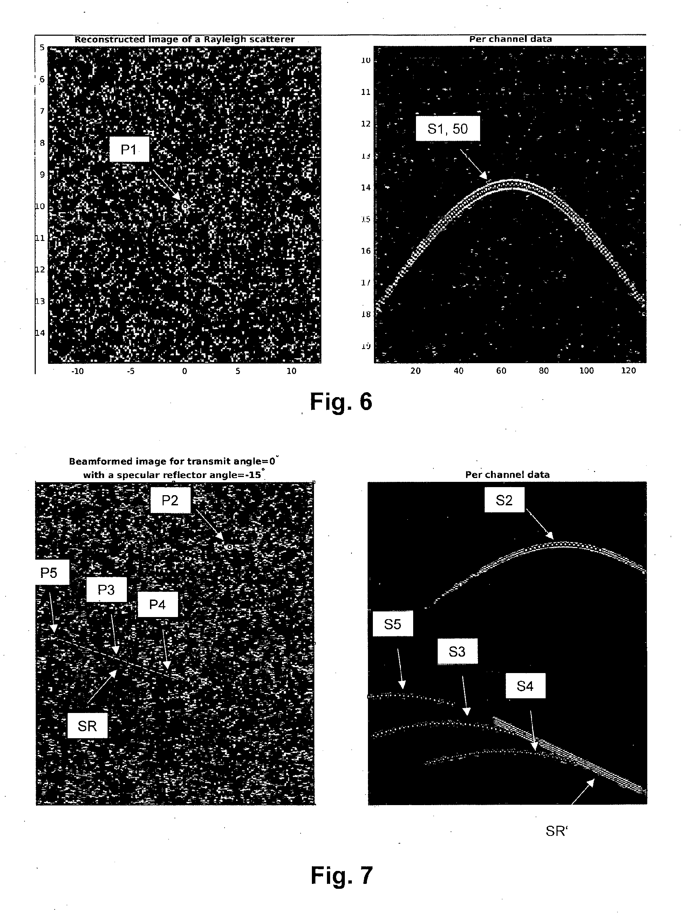

For instance, in the example of fig. 6 the maximum of the correlation r(P, $\gamma$) locates at $\gamma$ = -10°.

The operations described above may be applied at each pixel of the final images in order to have specular information on the whole medium. Because doing all the detection steps for each pixel requires a lot of computations, it is possible to implement the algorithm on for example a GPU in order to accelerate it and build a whole image in acceptable time. The specular beamforming may detect specular reflectors in the medium and their angles. It may also be a way to build B-Mode images in an optimized way.

The specular information may improve a DAS beamforming for both synthetic and conventional B-Mode. Conventionally, larger receive apertures of a transducer device may be used because of the assumption of Rayleigh scatterers, to maximize the signal coming from the reflector, but also to be sure to capture all the useful echoes because there is no preconception about the medium. A conventional beamforming process is rather not adapted for specular reflectors be-cause their echoes have a privileged direction following Snell's law. Depending on their tilt angle, planar reflectors are badly reconstructed and only a limited part of the receive aperture bears the specular signal. However, thanks to the improved image construction process of the present disclosure, one knows whether a reflector is specular or not, and its tilt angle. It is now possible to adapt the DAS reconstruction.

Fig. 6 to Fig. 9 shown an example of how a process of image construction (i.e. a beamforming process) may be optimized according to the present disclosure.

Fig. 6 shows an example of a conventionally reconstructed image of a Rayleigh scatterer (left) and the associated spatio-temporal signal data (right).

In more detail, the left diagram in fig. 6 shows image data with a plurality of pixels constructed by a conventional image construction process (for example according to a B-mode or a synthetic B-mode). The horizontal axis of the left diagram corresponds to the x-axis in fig. 1, i.e. to an axis extending along the transducer array and/or along the surface of the medium (on which the transducer device may be placed). The vertical axis may denote the z-axis in fig. 1, i.e. the depth direction of the medium. In the present example, the medium may comprise a Rayleigh scatterer P1, as visible in the reconstructed image data.

The right diagram in fig. 6 shows associated spatio-temporal signal data. The horizontal axis of the right diagram corresponds again to the x-axis in fig. 1, i.e. to an axis extending along the transducer array and/or along the surface of the medium (on which the transducer device may be placed). More in particular, the horizontal axis may denote the single channels of the respective transducer elements. The vertical axis may denote the temporal dimension of the collected spatio-temporal signal data.

In the present example, the medium may comprise a Rayleigh scatterer P1, as visible in the reconstructed image data on the left side of fig. 6.

Accordingly, the spatio-temporal signal data comprise a plurality of signals respectively received by the plurality of transducer element elements during a period of time. A specific subset S1 of spatio-temporal signal data is schematically illustrated along a line 50. The subset may correspond to signal data selected by a conventional DAS beamforming process to reconstruct the pixel (or pixels), which represent the Rayleigh scatter P1 in the left diagram of Fig. 6.

Accordingly, the dotted line 50 may be theoretical line and/or an imaginary line. In other words, the sections of the signals covered by the dotted line may be associated with the spatial region.

The dotted line 50 may be precomputed according to the DAS beamforming process.

As shown in the example of fig. 6, a conventional DAS beamforming process may correctly reconstruct a scatterer with a typical size < wavelength. Hence, the scatterer P1 may be suitably illustrated in the left diagram. For such Rayleigh scatterers, the receive angle may namely be considered to be namely equal to the transmit angle of an emitted ultrasound beam.

Fig. 7 shows an example of a conventionally reconstructed image of a Rayleigh scatterer and a specular reflector (left) and the associated spatio-temporal signal data (right).

In this example, a specular reflector SR, whose position is indicated by a dashed white line in the left diagram, may have a tilt angle of 15° with respect to the x-axis. The transmit angle of an emitted ultrasound beam (i.e. of a planar wave for synthetic B-mode) may be 0°. When the specular reflector is insonified by the wave, the backscattered field has a different pattern than that one of a Raleigh scatterer (as for example scatterer P2). Conventional receive delay laws of a conventional DAS beamforming process cannot permit to reconstruct these reflectors correctly. The exemplary pixels P3, P4 and P5 lying on the specular reflector SR may have respective subsets S3, S4 and S5 in the spatio-temporal signal data of the right diagram. However, due to the specular reflectivity the subsets do not correctly cover the signal data which actually relate to spatial position SR' of the specular reflector SR. Hence, the specular reflector SR is not even visible in the image data.

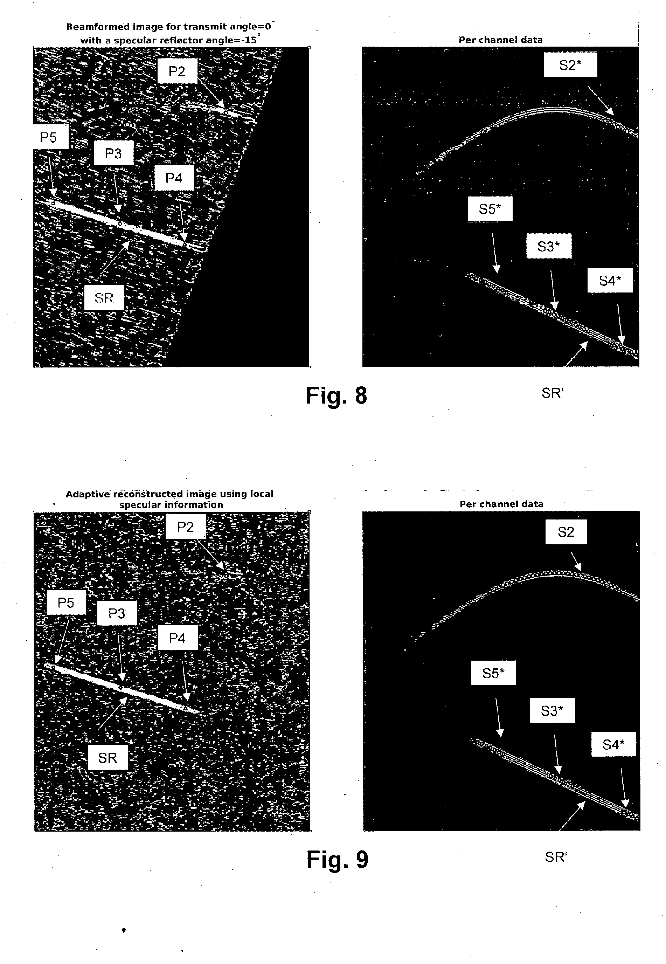

Fig. 8 shows the example of fig. 7, wherein the receive angle is adjusted according to the tilt angle of the specular reflector. As shown in fig. 8, the receive angle may be adapted according to Snell's law. Also the receive aperture size may be reduced. Performing this optimization operation gives the image data of the left diagram in which the specular reflector SR is now visible.

Hence, with the knowledge of the specular angle, the receive delay law may be tilted with respect to Snell's law. Because the delay law is aligned with the echo, only the apex of the parabola contains specular signal, and the edges of the delay law may be removed and/or ignored. Now, it is possible to use a reduced aperture, that is an increased f#, without any loss of signal. It can increase the contrast and the SNR because only the useful signal is summed, and it decreases the computation complexity thanks to the reduced aperture. '

However, since the above-described adjustment is also applied to the signal data S2* of the Rayleigh scatterer, this Rayleigh scatterer is not well reconstructed anymore. Therefore, the method may further be ameliorated, as illustrated in fig. 9.

Fig. 9 shows the example of fig. 7 and fig. 8, wherein the receive angle is adjusted according to the tilt angle of the specular reflector, but only for the pixels illustrating the specular reflector. Accordingly, the receive delay law and receive aperture may be adapted for any pixel, knowing if this pixel is part of a Rayleigh scatterer or a specular reflector. In case of a Rayleigh scatterer, no optimisation is necessary.

As demonstrated by the example of fig. 6 to 9, the method according to the present disclosure may be applied on the DAS beamforming of a synthetic B-mode. In synthetic B-mode (i.e. using plane emission waves) a specular reflector of a medium may be better reconstructed in the image data. For example, a hand tendon may not be reconstructed with a narrow aperture when Snell's law is not followed (i.e. the method of the present disclosure is not applied), but may appear clearly when the delay laws are aligned with the specular reflection according to the method of the present disclosure.

The method according to the present disclosure may also be applied on the DAS beamforming of a conventional focused B-Mode (i.e. using focalized emission waves). If the likelihood of having a specular reflector is high, the specular angle map may be given as an input of the DAS beamforming. Then the beamforming may use an adapted receive angle following Snell's law for each pixel, according to the method of the present disclosure. Again, a reduced aperture may be sufficient for the DAS beamforming of specular parts because the delays laws are now tilted with respect to the echoes.

As already described, in order to build such an image, it is desirable to insonify the medium first with plane waves to compute the specular likelihood and angle maps, and then to use focused beams to construct the conventional B-Mode image data. One way to improve the efficiency of such a beamforming would be to adapt the size of the aperture to the type of the reflector lying at each pixel. If, according to the likelihood map, it is a judged to be specular reflector (for example a likelihood above a predefined threshold), a reduced aperture may be used but tilted with respect to Snell's law. On the contrary, large apertures may be used for the other pixels (i.e. pixels with relatively low specular likelihood and/or below the predefined threshold) because the pixel might be located on a Rayleigh reflector.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

The terms "record" and "receive" may be used synonymously throughout this disclosure unless denoted differently.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

**Claims**

1. A method of optimizing a process for constructing ultrasound image data of a medium, comprising:

   providing (a) ultrasound spatio-temporal signal data of the medium,
   determining (c) a specular property of the medium as a function of the signal data,
   optimizing (d) the process based on the specular property.

2. The method according to claims 1, wherein
   determining (c) the specular property comprises:

   determining for a spatial region in the medium a probability of presence of a specular reflector and/or a specular reflector angle of the specular reflector, or
   determining (c) the specular property comprises:

   determining (c1) for each of a plurality of spatial regions in the medium a probability of presence of a specular reflector and/or a specular reflector angle of the specular reflector, and
   constructing (c2) a specular property map based on the determined probabilities and/or specular reflector angles.

3. The method according to claims 1 or 2, wherein
   the spatio-temporal signal data comprise at least one of:

   radio-frequency, RF, signal data,
   IQ demodulated data (what is used in practice),
   pre-beamformed ultrasound data,
   multichannel signal data,
   signal data obtained by a transducer device comprising a plurality of transducer elements, wherein the output of each transducer element forms a channel of the signal data.

4. The method according to any one of the preceding claims, wherein
   the process comprises a beamforming process, wherein ultrasound signal data of the medium is beamformed to obtain image data of the medium.

5. The method according to any one of the preceding claims, wherein
   the process is a B-mode imaging process or synthetic B-mode imaging process, and/or the image data is B-mode image data.

6. The method according to any one of the preceding claims, wherein
   the signal data used in the process and/or for beamforming at least partially corresponds to the signal data used for determining the specular property.

7. The method according to any one of the preceding claims, wherein
   the process and/or the beamforming process comprises:

   determining a subset of signal data as a function of a predefined spatial region in the medium, and
   determining image data of the spatial region based on the subset.

8. The method according to any one of the preceding claims, wherein
   the subset comprises a spatial and temporal selection of signal data.

9. The method according to any one of the preceding claims, wherein

   the spatial selection defines a receive aperture and/or a selection of channels used for forming the subset, and/or
   the temporal selection is based on a predefined delay algorithm.

10. The method according to any one of the preceding claims, wherein
    the process is optimized by adapting (d1) the subset of signal data and/or the spatial and temporal selection as a function of the specular property.

11. The method according to any one of the preceding claims, wherein

the spatial and temporal selection is further determined as a function of an estimated receive angle associated with a response signal received from the predefined spatial region, wherein
the spatial and temporal selection is adapted (d1) by changing the receive angle as a function of the determined specular reflector angle of a specular . reflector identified at the spatial region.

12. The method according to any one of the preceding claims, wherein
the spatial and temporal selection is adapted (d1, d2) by changing and/or limiting the spatial selection as a function of the specular property.

13. The method according to any one of the preceding claims, wherein

the spatial and temporal selection is further determined as a function of a receive aperture defined by a selection of channels used to receive a response signal from the predefined spatial region, wherein
the spatial selection is changed and/or limited (d1, d2) by respectively changing and/or reducing the aperture.

14. The method according to any one of the preceding claims, wherein
the process is optimized only for those subsets which are associated with a spatial region having a probability of presence of a specular reflector above a predefined threshold

15. The method according to any one of the preceding claims, wherein

ultrasound signal data of the medium is associated to a plurality of ultrasound waves emitted in the medium, wherein
the emitted ultrasound waves comprise non-focalized and/or plane waves having different emission angles.

16. A method of constructing ultrasound image data of a medium, comprising:

a method according to any one of the preceding claims, and
constructing (e) ultrasound image data of the medium using the optimized process.

17. A computer program comprising computer-readable instructions which when executed by a data processing system cause the data processing system to carry out the method according to any one of preceding method claims.

18. A system for optimizing an ultrasound process for constructing ultrasound image data of a medium, wherein the system comprises a processing unit configured to:

receive (a) ultrasound spatio-temporal signal data of the medium,
determine (c) a specular property of the medium as a function of the signal data,
optimize (d) the process based on the specular property.

**Fig. 1**

**(a): Provide ultrasound spatio-temporal signal data of the medium**

> (a1) (optional): Transmit a pulse into the medium

> (b2) (optional): Receive in response the plurality of signals from the medium

**(b): (optional) Provide ultrasound signal data of the medium for the process of constructing image data**

> (b1) (optional): Transmitting a pulse into the medium

> (b2) (optional): Receive in response the plurality of signals from the medium

**(c): Determine a specular property of the medium**

> (c1) (optional): determine for each of a plurality of spatial regions in the medium a probability of presence of a specular reflector and/or a specular reflector angle of the specular reflector

> (c2) (optional): constructing a specular property map based on the determined probabilities and/or specular reflector angles

**(d): Optimize the imaging process**

> (d1) (optional): Adapt a receive angle (i.e. shift the relative position of the receive aperture on the transducer array)

> (d2) (optional): Adapt (reduce) the receive aperture

**(e) (optional): Construct ultrasound image data of the medium using the optimized process**

**Fig. 2**

Fig. 3

Fig. 4

Fig. 5

EP 4 321 900 A1

Fig. 6

Fig. 7

Beamformed image for transmit angle=0
with a specular reflector angle=-15°

P2
P5
P3
P4
SR

Per channel data

S2*
S5*
S3*
S4*
SR'

Fig. 8

Adaptive reconstructed image using local
specular information

P2
P5
P3
P4
SR

Per channel data

S2
S5*
S3*
S4*
SR'

Fig. 9

Europäisches
Patentamt

European
Patent Office

Office européen
des brevets

# EUROPEAN SEARCH REPORT

Application Number

EP 22 31 5181

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/138403 A1 (BANDARU RAJA SEKHAR [NO] ET AL) 7 May 2020 (2020-05-07) <br> * abstract; figure 2 * <br> * paragraphs [0033], [0037] – [0039] * <br> * paragraph [0053] – paragraph [0058] * <br> * paragraph [0060] – paragraph [0063] * <br> * claim 1 * | 1-18 | INV. <br> G01S7/52 <br> G01S15/89 <br> A61B8/08 <br> G10K11/34 |
| X,D | RODRIGUEZ-MOLARES ALFONSO ET AL: "Specular Beamforming", IEEE TRANSACTIONS ON ULTRASONICS, FERROELECTRICS, AND FREQUENCY CONTROL, IEEE, USA, vol. 64, no. 9, 1 September 2017 (2017-09-01), pages 1285-1297, XP011659657, ISSN: 0885-3010, DOI: 10.1109/TUFFC.2017.2709038 [retrieved on 2017-08-28] <br> * abstract; figure 15 * <br> * section III, last paragraph * <br> * section VI, first & second paragraph * <br> * section V, last paragraph * <br> * equations (16) and (21) * <br> * appendix B * | 1-18 | |
| X | WO 2015/153189 A1 (GEN ELECTRIC [US]) 8 October 2015 (2015-10-08) <br> * abstract * <br> * paragraphs [0034], [0036], [0041] * <br> * paragraphs [0042], [0044], [0047] * <br> * paragraphs [0052], [0057], [0059] * | 1-9, 15-18 | TECHNICAL FIELDS SEARCHED (IPC) <br><br> A61B <br> G01S <br> G10K |

The present search report has been drawn up for all claims

| Place of search | Date of completion of the search | Examiner |
|---|---|---|
| Munich | 10 January 2023 | Knoll, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 31 5181

This annex lists the patent family members relating to the patent documents cited in the above-mentioned European search report.
The members are as contained in the European Patent Office EDP file on
The European Patent Office is in no way liable for these particulars which are merely given for the purpose of information.

10-01-2023

| Patent document cited in search report | Publication date | Patent family member(s) | Publication date |
|---|---|---|---|
| US 2020138403 A1 | 07-05-2020 | US 2017086781 A1 <br> US 2020138403 A1 | 30-03-2017 <br> 07-05-2020 |
| WO 2015153189 A1 | 08-10-2015 | CN 106456124 A <br> JP 6576947 B2 <br> JP 2017509429 A <br> KR 20160140858 A <br> US 2015272549 A1 <br> WO 2015153189 A1 | 22-02-2017 <br> 18-09-2019 <br> 06-04-2017 <br> 07-12-2016 <br> 01-10-2015 <br> 08-10-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2101191 A2

**Non-patent literature cited in the description**

- Adaptive beamforming based on Snell's law of reflection. **RODRIGUEZ-MOLARES, A ; FATEMI, A ; TORP, H. ; LØVSTAKKEN, L.** In 2016 IEEE International Ultrasonics Symposium (IUS). IEEE, September 2016, 1-4

- **RODRIGUEZ-MOLARES, A ; FATEMI, A ; LØVSTAKKEN, L. ; TORP, H.** Specular beamforming. *IEEE Transactions on Ultrasonics, Ferroelectrics, and Frequency Control,* 2017, vol. 64 (9), 1285-1297